# EUROPEAN PATENT SPECIFICATION

(11) **EP 2 321 052 B1**
(45) Date of publication and mention of the grant of the patent: **25.09.2019**
(21) Application number: 09805632.8
(22) Date of filing: 07.08.2009
(51) Int. Cl.: G01N 21/77, A61M 39/18, C12M 1/34

(54) **DEVICE FOR EXPOSING A SENSOR TO A CELL CULTURE POPULATION IN A BIOREACTOR VESSEL**
VORRICHTUNG ZUR EXPOSITION EINES SENSORS EINER ZELLKULTURPOPULATION IN EINEM BIOREAKTORGEFÄSS
DISPOSITIF POUR EXPOSER UN CAPTEUR À UNE POPULATION DE CULTURE CELLULAIRE DANS UNE CUVE DE BIORÉACTEUR

(30) Priority: 08.08.2008 US 87579 P
(43) Date of publication of application: 18.05.2011
(73) Proprietor: BROADLEY-JAMES CORPORATION, Irvine, CA 92618 (US)
(72) Inventor: BROADLEY, Scott, T., Los Angeles CA 90028-6218 (US); GARRAHY, Robert, J., Villa Park CA 92861-2228 (US)
(74) Representative: Gyi, Jeffrey Ivan
(86) International application number: PCT/US2009/053215
(87) International publication number: WO 2010/017519

(56) References cited:
- DE-U1-202005 011 177
- IL-A- 88 967
- JP-A- 2003 149 137
- US-A- 4 403 984
- US-A- 5 096 671
- US-A- 5 128 019
- US-A1- 2005 239 199
- US-A1- 2005 242 296
- US-A1- 2006 051 874
- US-A1- 2007 185 472
- US-A1- 2007 252 290

## Description

### RELATED APPLICATIONS

This application claims the benefit of U.S. Provisional Application No. 61/087,579 filed August 8, 2008.

### BACKGROUND OF THE INVENTION

### Field of the Invention

This application generally relates to devices and methods for use with disposable bioreactor systems. More particularly, this application relates to disposable probes for exposing a cell culture population to fluorescence dots.

### Description of the Related Art

Bioreactor systems ("bioreactors") or fermenters include containers which are used for fermentation, enzymatic reactions, cell culture, tissue engineering, and food production, as well as containers used in the manufacture of biologicals, chemicals, biopharmaceuticals, microorganisms, plant metabolites, and the like. Bioreactors vary in size from benchtop systems to large stand-alone units. The containers or "vessels" used in bioreactor systems can vary in size from less than about one (1) liter to about one thousand (1000) liters or more. The stringent asepsis requirements for sterile production in some bioreactors can require elaborate systems to achieve the desired product volumes. Consequently, the production of products in aseptic bioreactors can be costly which provides the motivation for pursuing improved systems. Pre-sterilized disposable bioreactor systems or "bioreactor bags" have been developed that need not be cleaned, sterilized or validated by end users, all of which can lower production costs.

Current practice for monitoring cell culture populations in standard glass and steel bioreactors involves introducing applicable probes, such as temperature, pH, or dissolved oxygen or dissolved carbon dioxide probes, through a port in the reactor wall or head plate. Some systems incorporate optical-based probes and other devices for measuring dissolved oxygen, pH, and dissolved CO₂. However, current probes and devices do not address significant issues related to sterilization requirements, sensor disposability and cost, and sensor shelf life which make the current devices less than optimal, especially when used in conjunction with a disposable bioreactor bag. Several applications variously describe an arrangement of a sensor that is provided in relation to a vessel or bag, such as US 2007/185472, 2007/0252290 and US 2006/0051874.

### SUMMARY OF CERTAIN EMBODIMENTS

The system, method, and devices of the invention as defined in the claims each have several aspects, no single one of which is solely responsible for its desirable attributes. Without limiting the scope of this invention, its more prominent features will now be discussed briefly. After considering this discussion, and particularly after reading the section entitled "Detailed Description of Certain Embodiments" one will understand how the features of this invention provide advantages over other sensors.

Embodiments of a sensor well that can be used with a bioreactor vessel, including a disposable bag, are illustrated and described herein. In one embodiment a sensor well for use with a bioreactor vessel includes a sheath; a sensing element disposed on or in a portion of the sheath; a signal transmitter comprising a waveguide disposed within at least a portion of the sheath and configured to provide signals to and/or receive signals from the sensing element and provide signals to and/or receive signals from a sensor controller; a connector configured to attach the sensor well to a portion of a bioreactor vessel, the connector comprising an aperture through which the sheath can be deployed into the bioreactor vessel; and a collapsible bellows which houses the sheath when in an undeployed position, the bellows coupled to one end of the sheath, the bellows, the connector, and the sheath configured to form at least a portion of a hermetically sealable and sterilizable enclosure, wherein deploying the sheath through the aperture in the connector exposes the sensing element is exposed to media contained in a bioreactor vessel while maintaining a sterile environment in the bioreactor vessel. The sensing element comprises fluorescing material and may be configured as a fluorescent dot. The sensor well can also include a plurality of sensing elements disposed on the sheath and a plurality of signal transmitters disposed at least partially within the sheath, each signal transmitter associated with a least one sensing element. In one embodiment, the sensing element includes an electronic sensor. In one embodiment, the sensing element includes an electronic sensor. The bellows can comprise a telescoping structure or a flexible material configured in an accordion-like structure. In some embodiments, the fluorescing material can be disposed on, or impregnated in, a sheath, for example a plastic sheath. In some embodiments, the fluorescing material is affixed to the sheath by a translucent material, having adhesive properties, such that the sensor is exposed to the media when deployed. The fluorescing material can be configured to indicate a characteristic or measurement of the cell culture to which it is exposed, including for example, pH, dissolved oxygen, or carbon dioxide. The sensing element can also be an electronic sensor which can be configured to sense, for example, temperature, conductivity, or osmolality. The signal transmitter can include wires, or other means to convey a signal from and/or to the sensing element.

A device for use with a bioreactor system is also provided. The device includes a waveguide; a sheath surrounding the waveguide; a sensing element disposed at a distal end of the sheath, the sheath and the sensing element configured to move between a first position and a second position; a sterilizable enclosure configured to protect the sensing element from an exterior environment at least when the sensing element and the sheath are in the first position; and a sterile connector disposed at a distal end of the enclosure, wherein the distal end of the sheath is disposed distal of the sterile connector when the sensing element and the sheath are in the second position. The sensing element includes a fluorescing material. In another embodiment, the sensing element includes one or more dots, each comprising fluorescing material. In some aspects, at least one of the dots is configured to fluoresce, when radiated, to indicate a characteristic of a cell culture population to which it is exposed. The one or more dots can be configured to fluoresce to indicate pH, dissolved oxygen content, or carbon dioxide content. In one embodiment, the sensing element is an electronic sensor. The sensing element can be configured to measure temperature, conductivity, or osmolality.

Also provided is a device for use with a bioreactor vessel. The device includes a sensor comprising a signal transmitter, a sheath, the sheath at least partially surrounding the signal transmitter, and at least one sensing element disposed at a distal end of the sheath, the sensor configured to move between a first and a second position; bellows surrounding at least a portion of the sensor at least when the sensor is in the first position; and a sterile connector disposed at a distal end of the bellows, wherein the bellows, the sterile connector, and the sheath form a sterilizable enclosure when the sensor is in the first position, and wherein the sensor is movable with respect to the connector such that the distal end of the sheath is disposed distal of the sterile connector when the sensor is in the second position. The signal transmitter includes an optical waveguide and in one embodiment, the sensing element includes a fluorescent dot. In another embodiment, the signal transmitter includes a communication wire.

In another embodiment, a device for use with a bioreactor vessel includes a sensor comprising at least one signal transmitter and at least one sensing element; and an enclosure defining a sterilizable space around the sensor, the enclosure comprising a connector configured to provide a sterile connection between the enclosure and a corresponding connector on a bioreactor vessel.

In yet another embodiment a device for monitoring media in a bioreactor bag having at least one port with a first sterile connector coupled thereto is provided. The device includes means for monitoring a characteristic of the media, the monitoring means comprising at least one fluorescent dot, means for transmitting light to the fluorescent dot, and means for transmitting a response signal from the fluorescent dot to a controller; means for protecting a sterile environment of the monitoring means; means for making a sterile connection between the monitoring means and the first sterile connector; and means for inserting the monitoring means into the bioreactor bag once a sterile connection has been made. The means for transmitting light includes an optical waveguide. The means for transmitting a response signal from the fluorescent dot to the controller comprises an optical waveguide.

Another embodiment includes a bioreactor system. The bioreactor system includes a disposable bioreactor bag comprising at least one port having a first sterile connector coupled thereto; a sensor comprising at least one signal transmitter, at least one sensing element, and a second sterile connector; and an enclosure defining a sterilizable space around at least the sensing element, the enclosure comprising a connector configured to provide a sterile connection between the enclosure and a corresponding connector on a bioreactor bag.

Another embodiment includes a method of monitoring media in a bioreactor vessel having at least one port with a first sterile connector coupled thereto. The method includes coupling the first sterile connector of the bioreactor vessel to a disposable sensor well, the sensor well including bellows, an injectable member, and a second sterile connector having a seal, the bellows, the injectable member, and the second sterile connector forming a sterilizable space therebetween, the injectable member comprising at least one waveguide and at least one fluorescent dot, the fluorescent dot being disposed in the sterilizable space before the injectable member is injected into the bioreactor vessel; removing the seal of the second sterile connector; and injecting the injectable member into the bioreactor vessel.

### BRIEF DESCRIPTION OF THE DRAWINGS

The above mentioned and other features of the invention will now be described with reference to the drawings. They are intended to illustrate and not to limit the invention. The drawings contain the following figures:
**FIGURE 1** is a cross sectional view of a sensor well shown in an undeployed position.
**FIGURE 2** is a cross-sectional view of another sensor well shown in an undeployed position and coupled to a bioreactor vessel.
**FIGURE 3** is a cross-sectional view of the sensor well of **FIGURE 2** in a deployed state.
**FIGURE 4** is a perspective view of the sensor well of **FIGURE 2****,** shown housed in a sterile package.
**FIGURE 5** a perspective view of the sensor well of **FIGURE 4****,** shown removed from the package and with its sealing layer removed.
**FIGURES 6A** through **6E** show cutaway perspective views of sensor wells configured in accordance with various embodiments.
**FIGURE 7** is a schematic drawing illustrating a portion of an exemplary bioreactor system which can be used with embodiments of the invention.
**FIGURE 8** is a schematic drawing illustrating a sensor well in use with the bioreactor system of **FIGURE 7****.**
**FIGURE 9a** is a side view of a sensor well in an undeployed state.
**FIGURE 9b** is a cross-sectional view of the sensor well of **FIGURE 9a** in an undeployed state, with the signal transmitter inserted into the sheath.
**FIGURE 9c** is a cross-sectional view of the sensor well of **FIGURE 9a** in an undeployed state, with the signal transmitter removed from the sheath.
**FIGURE 10a** is a side view of another sensor well in an undeployed state.
**FIGURE 10b** is a cross-sectional view of the sensor well of **FIGURE 10a** in an undeployed state, with the signal transmitter inserted into the sheath.
**FIGURE 10c** is a cross-sectional view of the sensor well of **FIGURE 10a** in an undeployed state, with the signal transmitter removed from the sheath.
**FIGURE 11** is a cross-sectional view of an embodiment of the sensor well that includes inlet and outlet ports.

### DETAILED DESCRIPTION OF CERTAIN EMBODIMENTS

Reference will now be made in detail to the preferred embodiments of the invention, examples of which are illustrated in the accompanying drawings. While the invention will be described in conjunction with certain preferred embodiments illustrated and described herein, it will be understood that they are not intended to limit the invention to those embodiments. On the contrary, the invention is intended to cover alternatives, modifications and equivalents, which may be included within the scope of the invention as defined by the appended claims. In addition, features of the invention that are described in one embodiment are not limited to that embodiment unless specifically stated as such; instead, certain features may be suitably used in the other embodiments described herein or other embodiments of the invention that are not specifically described.

Some existing optical sensing systems designed for use with disposable bioreactor bags may include sensing material, for example, fluorescing material, which is incorporated into the design of the bioreactor bag. Such designs present a number of disadvantages. For example, to meet sterilization requirements, to that embodiment unless specifically stated as such; instead, certain features may be suitably used in the other embodiments described herein or other embodiments of the invention that are not specifically described.

Some existing optical sensing systems designed for use with disposable bioreactor bags may include sensing material, for example, fluorescing material, which is incorporated into the design of the bioreactor bag. Such designs present a number of disadvantages. For example, to meet sterilization requirements, some sensors are incorporated into the bag and sterilized with the bag, and then supplied to a customer as a bioreactor bag having a certain sensor. In preferred embodiments, the sensor well comprises materials that can be gamma ray sterilized. This can present logistics problems as well as increase costs, particularly for optical-based sensors which cannot be steam sterilized and must be irradiated instead. Also, bioreactor bags have a certain shelf life (for example, about eighteen (18) months). Fluorescence material used in many optical sensors also has a shelf life that is typically shorter than the bioreactor bags (for example, about one year). Accordingly, the expiration of fluorescence material incorporated into a disposable bioreactor bag can shorten the shelf life of the bioreactor bag. In addition, certain calibration information is associated with the fluorescence material. The calibration information must be available when using the bag to ensure correct readings of the fluorescence material. Accordingly, a sensor containing fluorescence material incorporated into the bag requires that the sensor calibration data always "travel" with the bag, which can increases logistics problems and expenses.

Embodiments of the invention desirably provide a sensor well for use with a bioreactor vessel or container. The sensor well is configured to maintain a sterile environment around sensing components which can include one or more optical or electronic sensors. The sensor well is configured such that it can be connected, in a sterile manner, to a sterile bioreactor vessel before or after the vessel is provided from the vessel manufacturer and still maintain the sterile environment inside the vessel. This is one of the advantages of this sensor well. This allows, for example, a user to select a particular bioreactor bag from stock and then configure the bag with a particular sensor well, if desired. In other words, the sensor well can be provided to a customer packaged and sterilized, and the customer can store the sensor well and introduce it into a bioreactor vessel when desired or deemed necessary. Calibration data relating to the sensor well can be packaged and stored with the sensor well so that it is readily available when the sensor well is installed into a bioreactor vessel. The sensor well configuration allows it to be connected while the vessel is in use so that a sensor can be deployed in the bioreactor vessel and still maintain a sterile environment inside the vessel, so long as the bioreactor vessel is suitably configured with a connector for mating with a matching connector of the sensor well. The embodiments of the sensor well generally described herein are described for use with a disposable bioreactor bag, but the embodiments should not be construed as limited to such use. Instead, embodiments of the sensor well can be equally used with a hard-walled bioreactor vessel, for example, one made from steel or glass.

Specifically, embodiments illustrated and described herein provide a sensor well which can be introduced into a sterile environment for example in a single-use disposable vessel of a bioreactor system or another type of bioreactor system. Various embodiments can be configured with one or more sensing element so as to sense one or more desired characteristics of media in a bioreactor vessel. The sensor well can include one or more of many types of sensors, including but not limited to optical sensors including fluorescing sensors, potentiometric ion-selective sensors, amperometric sensors, and resistive sensors. In addition, embodiments advantageously provide a sensor whose calibration data remains with the sensor well, separate from the bioreactor bag or other vessel with which the sensor is used, so the particular sensor well can be calibrated into any vessel the sensor is placed in when it is actually placed in operation.

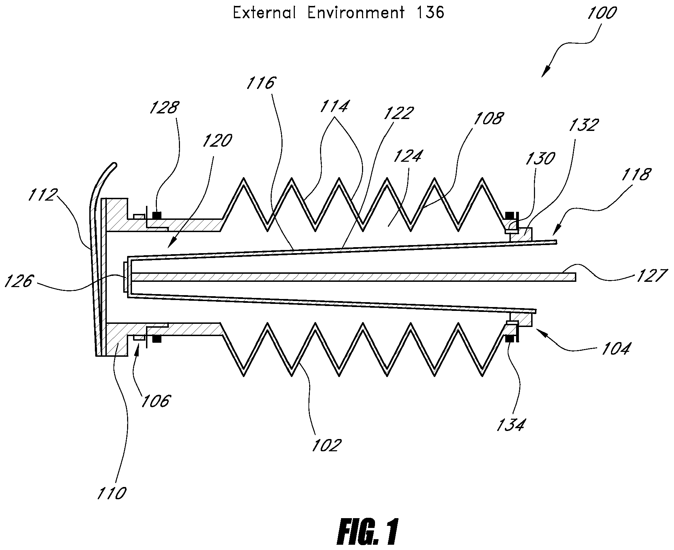

**FIGURE 1** illustrates a sensor well **100** shown in **FIGURE 1** in an undeployed state. The sensor well **100** includes an enclosure **102** having a proximal end **104,** a distal end **106,** and an inner surface **108.** A connector **110** is disposed at the distal end **106** of the enclosure **102.** A removable sealing layer **112** covers the distal end **106** of the enclosure **102** and hermetically seals the enclosure **102.** The enclosure **102** also includes one or more bellows **114** disposed between the proximal end **104** and the distal end **106.** The bellows **114** are configured to be compressible, such that the proximal end **104** of the enclosure **102** can be moved closer to the distal end **106** of the enclosure **102.**

The sensor well **100** also includes a sheath or injectable member **116** disposed substantially within the enclosure **102.** In **FIGURE 1** the sensor well **100** is in an undeployed state such that the sheath is still within the enclosure **102.** The sheath **116** has a proximal end **118,** a distal end **120,** and an outer surface **122.** Together, the inner surface **108** of the enclosure **102** and the outer surface **122** of the sheath **116** form a sterilizable space **124** within the enclosure **102.** A sensing element **126** is disposed at the distal end **120** of the sheath **116,** inside the enclosure **102.** The sensing element **126** communicates with a signal transmitter **127** disposed inside the sheath **116,** outside of the sterilizable space **124.** The sheath **116,** sensing element **126** and the signal transmitter **127** are sometimes collectively referred to herein as the "sensor." The sheath **116** is coupled to the proximal end **104** of the enclosure **102** such that the sheath **116** can move with the proximal end **104** when the proximal end **104** is moved toward the distal end **106** of the enclosure **102.** By such a configuration, the well **100** can be provided to a user in an undeployed and pre-sterilized state, for example, inside a hermetically sealed plastic bag with the sealing layer **112** sealing the opening in the connector **110.** The user can then couple the well **100** to a suitable connector, such as a sterile connector disposed on a bioreactor bag, remove the sealing layer **112** and an associated sealing layer on the connector disposed on the bioreactor bag, and deploy the sheath **116** into the bioreactor bag without exposing the sensing element **126** or the interior of the bioreactor bag to an unsterile environment. In addition, the user can replace the signal transmitter **127** without necessarily removing the well **100** from the bag because the proximal end **118** of the sheath may be open or exposed to the environment outside the bag allowing access to components in the sheath.

In **FIGURE 1**, the connector **110** is attached to the enclosure **102** and forms a part of the enclosure **102.** The connector **110** can be held in place by a clamp (or securing band) **128** which at least partially surrounds the joint between the connector **110** and the enclosure **102.** In this embodiment, a threaded insert **130** and a locking nut **132** cooperate to secure the sheath **116** at the proximal end **104** of the enclosure **102.** An additional clamp **134** can optionally be included to surround the joint between the sheath **116** and the proximal end **104** of the enclosure **102** and provide additional securement. Although not illustrated, one or more o-rings or other types of seals can be included at this joint to provide an airtight seal at this joint. Of course, any suitable means can be used to couple the sheath **116** to the enclosure **102,** so long as the means provides an airtight seal between the interior **124** of the sensor well **100** and the exterior environment **136.**

In some embodiments, the enclosure **102** can comprise a syringe plunger sheath, such as the Silicone Syringe Plunger Sheath which can be purchased from Qosina Corporation of Edgewood, NY. The illustrated embodiments show an enclosure **102** having bellows **114** having an accordion-like structure which is configured to compress and allow the sheath **116** to move from a first undeployed position to a second deployed position while maintaining a sterile environment inside the enclosure **102.** However, other embodiments of the invention can have any other suitable configuration of material to achieve this functionality while maintaining a sterile interior in the enclosure **102.** For example, some embodiments can include enclosures having a telescoping configuration. Other examples of bellows include other flexible material(s) that allow the sensor well to be adequately deployed into a bioreactor vessel.

In some embodiments, the connector **110** can be a Kleenpak™ Connector available from PALL Corporation of East Hills, NY. Of course, any other suitable connector configured to sterilely connect two fluid environments can also be used with embodiments of the invention.

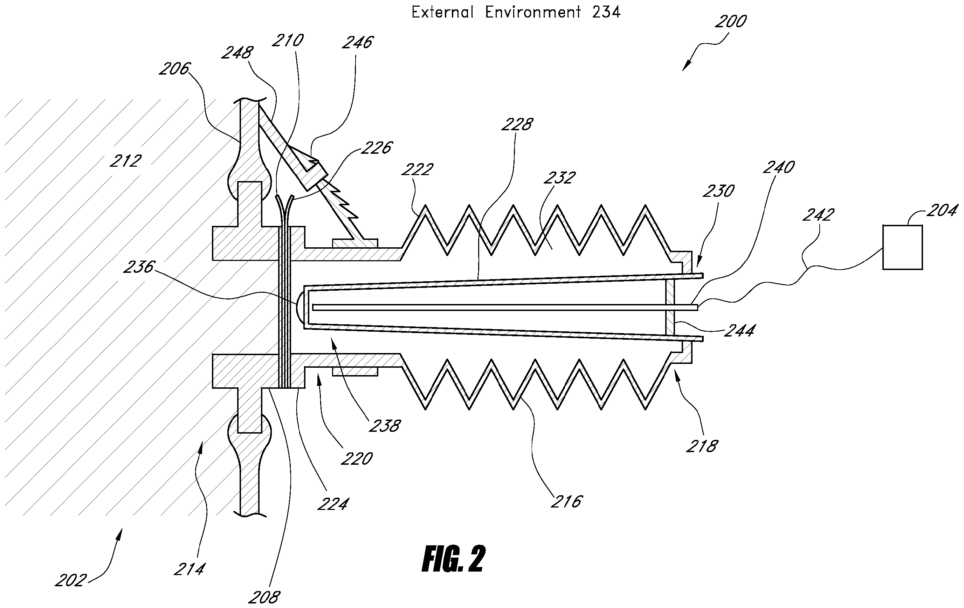

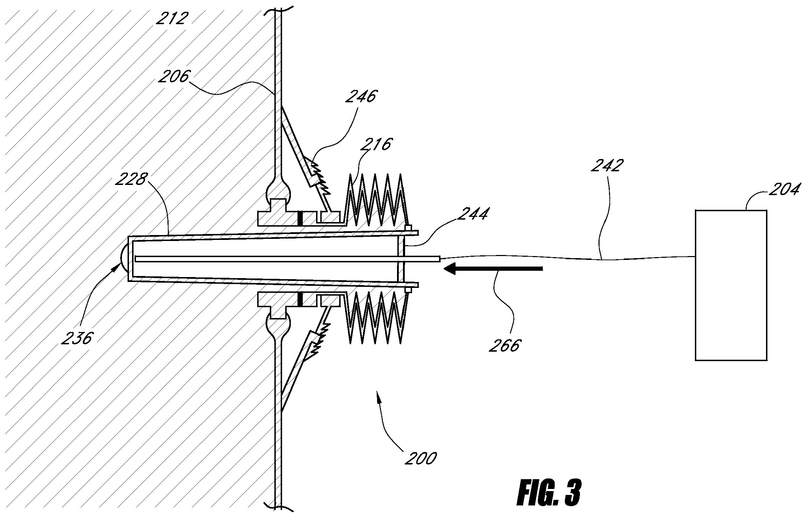

**FIGURES 2** and **3** illustrate the deployment of a sensor well **200.**

**FIGURE 2** shows the sensor well **200** in an undeployed state, coupled to a bioreactor system comprising a vessel **202** and a sensor controller (or processor) **204** disposed outside of the vessel **202** and as part of a bioreactor control system. The bioreactor control system includes a flexible bioreactor bag **206,** having at least one sterile connector **208** disposed on the bag **206.** The connector **208** is covered by a removable sealing layer **210.** A sterile environment is maintained in the bioreactor bag **206,** which when operable may be at least partially filled with media **212,** such as a cell culture population. The connector **208** is configured to allow the interior **214** of the bioreactor bag 206 to be fluidly connected to a second sterile environment, such as, for example, a fluid supply line, without exposing either the interior **214** of the bioreactor bag **206** or the second sterile environment to an unsterile environment.

The sensor well **200** includes an enclosure **216** having a proximal end **218,** a distal end **220,** and compressible bellows **222** disposed therebetween. A connector **224** is disposed at the distal end **220** of the enclosure **216,** and is covered by a sealing layer **226.** Disposed substantially within the enclosure **216** is a sheath **228.** The sheath **228** has a proximal end **230** which is sealingly coupled to the proximal end **218** of the enclosure **216** so as to separate an interior environment **232** of the enclosure **216** from the ambient, or external environment **234.** The sheath **228** is configured to move in a longitudinal direction as the bellows **222** are compressed (that is, as the proximal end **218** of the enclosure **216** is moved towards the distal end **220** of the enclosure **216**). For example, as illustrated in **FIGURE 2** the sensor well **200** is configured such that the sheath **228** moves horizontally to the left when deployed into a bioreactor vessel and moved from a first undeployed position to a second deployed position. A sensing element **236** is disposed at a distal end **238** of the sheath **228,** within the interior environment **232** of the enclosure **216.** In the illustrated example, the sensing element **236** comprises fluorescent material (for example, a fluorescent dot). The sensing element **236** can be configured to provide an indication of a characteristic of the media it is exposed to, for example, pH, dissolved oxygen, or dissolved carbon dioxide.

A fluorescent "dot" or "patch" is typically a section of polymer sheet that has been coated or impregnated with a fluorescent compound or mixture of compounds that fluoresce when excited by a proper light source. When excited by a light source with proper wavelength, the fluorescent compound fluoresces and then if the excited fluorescent compound encounters an analyte, the analyte affects (e.g., changes or quenches) the fluorescent signal. The fluorescence intensity or phase shift (decay time, preferred for reasons of system stability, precision and accuracy) measured by a spectrometer system is related to the analyte concentration.

Dot/patch sizes typically vary from a few millimeters to several centimeters in diameter. Thickness can vary from less than a quarter of a millimeter to several millimeters. The dot/patch can be covered with a layer of material to trap the fluorescent compound in the matrix of the dot/patch to prevent the fluorescent compound (for example, ruthenium complex or porphyrin) from leaching into the sample. An optical transparent adhesive can be used to attach the dot to different surfaces. The fluorescent compound can also be directly painted/applied on a supporting substrate when the there is no concern of leaching (such as when the fluorescent compound is chemically linked to the supporting substrate matrix).

Thus, fluorescent dots ("dots") as used herein, is a broad term that refers to a mass of material that is configured to have certain fluorescing characteristics (for example, to have a particular fluorescence to indicate pH, dissolved oxygen content, or CO₂ content), and that is disposed on a distal end of the sheath. In various applications, dots may be configured in various sizes (e.g., length, width, and height), shapes, colors, and compositions to exhibit desired sensing characteristics. Some examples of dot cross-sectional shapes includes circles, ovals, generally curvilinear shapes, squares, rectangles, triangles, generally polygonal shapes, and irregular shapes. In a configuration that includes numerous dots, the dots may be disposed in a uniform pattern or another pattern to produce a desired sensing effect, and each dot may each be about the same size or the dots can vary in size. A fluorescent dot can be three-dimensional or essentially two-dimensional, with very little or negligible thickness extending along a longitudinal axis of the sheath. In one embodiment, the thickness of the fluorescent dot is less than the width or length of the dot. In another embodiment, the dot has a relatively low profile at the distal end of the sheath.

A signal transmitter **240** is disposed at least partially within the sheath **228,** separated from the interior environment **232** of the enclosure **216** by the sheath **228.** The signal transmitter **240** is configured to transmit a signal to and/or from the sensing element **236** to the processor **204** via a connection line **242.** In the illustrated example, the signal transmitter **240** is an optical waveguide, configured to transmit a particular wavelength of light to illuminate the sensing element **236.** The signal transmitter **240** is configured to transmit a return signal from the sensing element **236** back to the processor **204.** A characteristic of the fluorescing material can be used to indicate a property of characteristic of media. For example, the wavelength or frequency of fluorescence, or the rate at which the fluorescence of the material decays can be used to determine a characteristic property of the bioreactor media **212,** such as, for example, pH, dissolved oxygen, or other characteristics of the media **212.** The distal end **238** of the sheath **228** can have a transparent window (not visible in **FIGURE 2**) so as to allow a signal to be transmitted to and from the sensing element **236** through the window. The signal transmitter **240** is held in place by a support, or sensor holder **244** disposed within the sheath **228.** In some embodiments, the signal transmitter **240** can be substantially disposed inside a sensor component enclosure within the sheath **228.**

As shown in **FIGURE 2****,** the connector **224** of the sensor well **200** is configured to mate with the connector **208** of the bag **206.** The sensor well 200 and/or the bag **206** can include one or more features configured to aid in securement of the well **200** to the bag **206** when the connectors **224**, **208** are coupled together. For example, the sensor well **200** can include a tether, or sensor well support, such as the illustrated ratcheted tether **246,** coupled to the distal end **220** of the well **200.** The tether **246** is configured to cooperate with a corresponding support **248** on the bioreactor bag **206** to support the well **200** when it is connected to the bag **206.**

**FIGURE 2** illustrates the sensor well **200** connected to the bioreactor bag **206** with the sealing layers **210**, **226** intact and the sensor well in an undeployed position. In such a position, the distal end **238** of the sheath **228** is disposed proximal of the distal end **220** of the enclosure **216,** within the interior environment **232** of the well **200.** Once the sensor well **200** is connected to the bag **206,** the sealing layers **210**, **226** can be removed to expose the interior **214** of the bag **206** to the interior environment **232** of the enclosure **216.** In some configurations the sealing layers **210**, **226** are removed by pulling them together in a direction about orthogonal to the attached sensor well **200.** The proximal end **218** of the enclosure **216** can then be pushed toward the distal end **220** of the enclosure **216,** thereby compressing the bellows **222** and moving the distal end **238** of the sheath **228** past the connector **224** and into the bag **206.**

**FIGURE 3** illustrates the sensor well **200** in this deployed position inside the bag **206.** To deploy the sensor well **200,** a force is generally applied in the direction indicated by an arrow **266.** In the deployed position, the sensing element **236** is exposed to such the media, or bioreactor contents **212** inside the bioreactor bag **206** so that it may perform its sensing function. For example, the sensing element **236** can be placed in contact with, in proximity to, or placed in the same environment as the media such that the media affects the sensing element **236.** The aseptic condition within the bioreactor **206** is maintained, however, as the only exposure caused by insertion of the well **200** into the bag **206** is exposure to the pre-sterilized enclosure **216,** the sheath **228,** and the sensing element **236.** Although not illustrated, the sensor well **200** and/or the bag **206** can be provided with one or more features to releasably or fixedly secure the well **200** in the deployed position. Such features can include any suitable means for achieving this function, including one or more tethers, clamps, or latches.

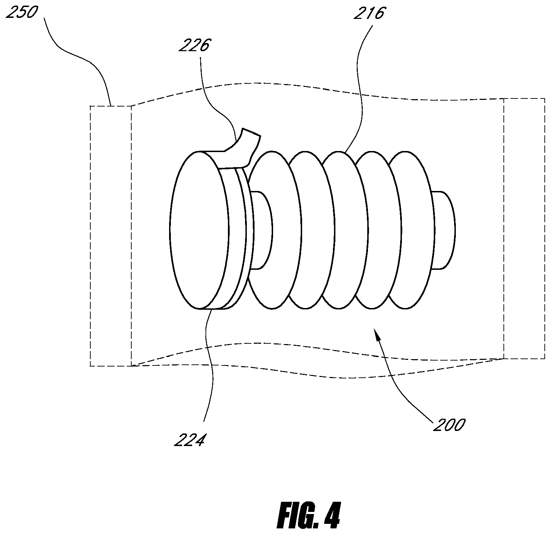

**FIGURE 4** shows a perspective view of the sensor well **200** as it can be provided to the user, pre-sterilized and sealed within a sterile package **250.** The sensor well **200** can be delivered with seal **226** in place such that the sensor well **200** is ready for use upon opening the package. Embodiments of the invention can be sold, transported, and stored in the sterile package **250,** and, thus, the sterility of the sensor well **200** can be maintained until it is needed. In embodiments in which the sensor element (not shown) comprises a light-sensitive element such as a fluorescent dot, the sterile package **250** can be opaque so as to protect the sensor element from degradation to ambient radiation. Additionally, the configuration of the enclosure **216,** in cooperation with the sealing layer **226** of the connector **224,** maintain the sterility of the internal environment of the enclosure **216** until such time as the sensor well **200** is coupled to a mating connector on a bioreactor vessel.

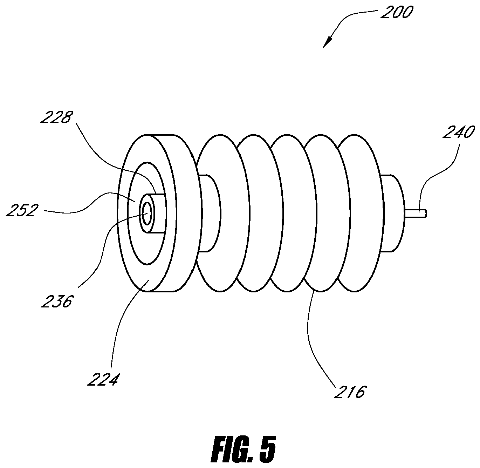

**FIGURE 5** shows a perspective view of the sensor well **200** removed from the package **250.** Here, the sealing layer **226** is shown removed to better illustrate the sensing element **236,** parts of the sheath **228,** and the interior **252** of the enclosure **216.**

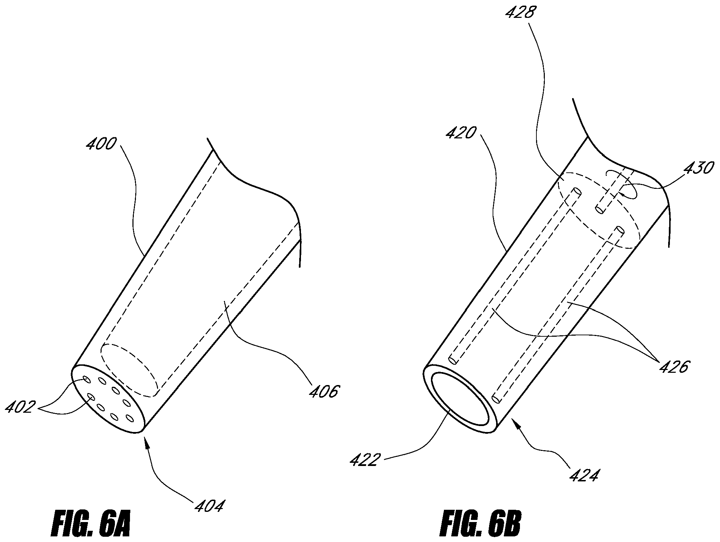

With reference now to **FIGURES 6A** through **6E****,** various configurations of sensors and sheaths are illustrated in accordance with embodiments of the invention. **FIGURE 6A** shows a sheath **400** having a plurality of sensing elements **402** disposed at a distal end **404** thereof. The sensing elements **402** can comprise fluorescent material configured as dots disposed in a spaced-apart fashion on the exterior surface of the distal end **404** of the sheath **400.** Inside the sheath **400,** and shown in dashed lines, is a signal transmitter **406,** which may be an optical waveguide configured to transmit light both to and from the sensing elements **402.** Although not illustrated, the distal end **404** of the sheath **400** can include a transparent window on which the sensing elements 402 are disposed to allow light to pass to and from the sensing elements 402.

Thus, some embodiments comprise fluorescent dots disposed on the exterior surface of the distal end 404 of the sheath 400. As described in greater detail above, when the fluorescent compound is excited by a light source with proper wavelength, the fluorescent dot fluoresces. When the sheath 400 is deployed inside the bioreactor bag, the fluorescent dot is placed in contact with the media, or bioreactor contents, inside the bioreactor bag so that it may perform its sensing function. The bioreactor contents held inside the bag, for example a cell culture population, may contain an analyte. If the excited fluorescent compound encounters analyte when the sheath **400** is deployed inside the bioreactor bag, the analyte quenches the fluorescent signal. The fluorescence intensity or phase shift measured by a spectrometer system can then be related to the analyte concentration.

In some embodiments, multiple signal transmitters may be included in the sheath **400.** In such embodiments, each signal transmitter may be associated with one or more of the fluorescing dots. As shown in the **FIGURE 6A****,** in this embodiment the signal transmitter **406** is dimensioned such that a surface of the signal transmitter **406** can receive a signal from all of the sensing elements **402.** As will be appreciated by one skilled in the art, by using a plurality of sensing elements, a sensor well can be configured to detect several different properties, or several different ranges of the same property.

**FIGURE 6B** illustrates another exemplary embodiment of a sheath **420.** In this embodiment, sheath **420** comprises a single deposit of fluorescent material **422** which is disposed on the distal end **424** of the sheath **420.** The fluorescent material **422** substantially covers the distal end **424.** Disposed inside the sheath **420** are multiple waveguides, this particular example includes two relatively small waveguides **426.** The waveguides **426** are mounted on a disk **428** which, in turn, is connected to a shaft **430.** This system is configured such that rotation of the shaft **430** causes the waveguides **426** to rotate about the axis of the shaft **430,** exposing different areas of the fluorescent material **422** to each of the waveguides **426** at different times. The shaft **430** can be configured to rotate, translate, or achieve a combination of these motions. In addition, the shaft **430** can be configured to move continuously, intermittently at a predetermined rate, or when desired by a user. With such an embodiment, the user is able to move the waveguides to a different area of the fluorescent material **422** as the previously used portion deteriorates, without needing to recalibrate test equipment.

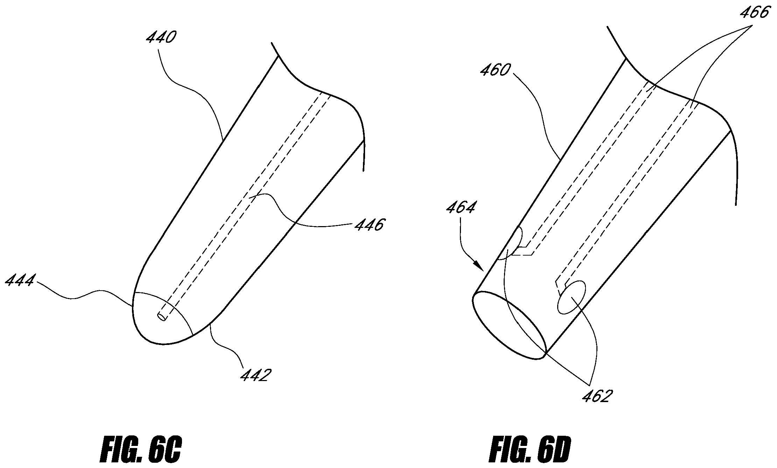

**FIGURE 6C** illustrates one of several tip geometries that may be incorporated with embodiments of the invention. A sheath **440** includes a generally bullet-shaped distal tip **442,** upon which a sensing element **444** is disposed. A signal transmitter **446** is disposed inside the sheath **440,** and is configured to communicate a signal, such as an optical signal, to and from the sensing element **444.** Of course, as will be apparent to one skilled in the art, embodiments of the present invention encompass a variety of alternative tip geometries in addition to the ones illustrated.

**FIGURE 6D** is an illustration of an embodiment comprising a sheath **460** having one or more sensing elements **462** located on a side of the sheath **460,** toward a distal end **464** of the sheath **460.** Such an embodiment can include one or more signal transmitters **466** disposed inside the sheath **460.** The signal transmitters **466** can extend substantially parallel to the longitudinal axis of the sheath **460** toward the distal tip **464,** and can curve or angle as they approach the distal tip so as to communicate with the sensing elements **462.** Embodiments can also include sensing elements disposed on the side of the sheath **460** and/or on the end of the sheath **460** even closer to the proximal end of the sheath.

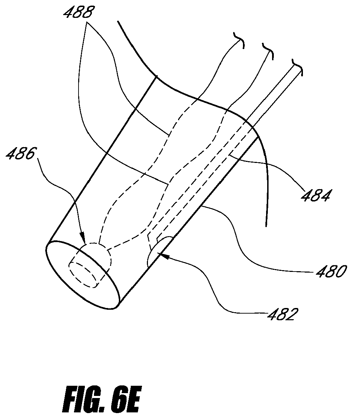

**FIGURE 6E** illustrates a sheath **480** according to a further embodiment of the invention. The sheath **480** includes multiple types of sensors, including an optical sensor comprising a fluorescing material **482** disposed on a side of the sheath **480,** and a signal transmitter **484** configured to communicate with the dot **482.** The sheath **480** also includes a second sensor comprising a sensing element **486** and a communication line, or wire **488.** It will be apparent to one skilled in the art that numerous embodiments of the invention can include multiple and diverse sensors in a single sheath.

Although the illustrated embodiments generally include an optical sensor comprising fluorescent material and a waveguide signal transmitter, embodiments of the invention can also include in addition a combination of other type of sensors such as a pH electrode or any other conventional pH or dissolved oxygen sensor, a thermal well, sensors configured to sense conductivity or osmolality, or any other type of sensor which is desirably placed in direct contact with media.

Persons of skill in the art will also understand bioreactor systems described herein can include multiple and diverse signal transmitters. For example, signal transmitters can also include communication lines or wires, and other means to convey a signal from and/or to the sensing element.

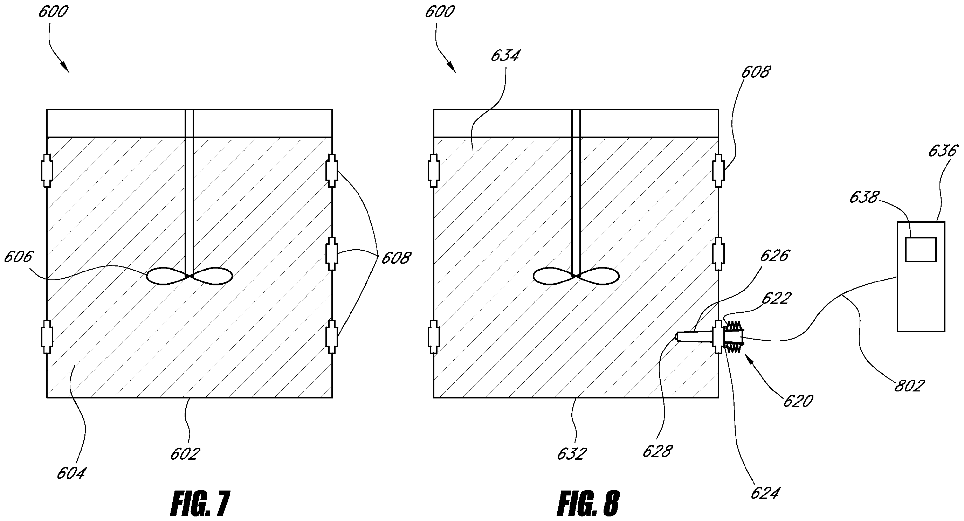

With reference now to **FIGURE 7****,** an exemplary bioreactor system 600 is illustrated with which the invention may be used. The system **600** comprises a bioreactor vessel **602,** which can be configured to be re-usable (for example, comprising metal or glass) or disposable. The vessel **602** is configured to maintain a sterile environment inside the vessel **602.** In operation, the vessel **602** can be at least partially filled with media **604.** The vessel **602** includes an agitator **606** configured to agitate the media **604.** In this embodiment the agitator **606** comprises a mechanical revolving structure but other agitators can also be used. The vessel **602** also includes one or more sterile connectors, or sterile ports **608** configured to allow fluid communication between the interior of the vessel **602** and a second fluid environment.

**FIGURE 8** illustrates a sensor well **620** deployed in a media filled vessel **632** of the bioreactor system **600.** The sensor well **620** includes an enclosure **622,** a connector **624,** and a sheath **626** with a sensing element **628** disposed at its distal end. The sensing element **628** provides a signal which is communicated to a processor **636** using a signal transmitter **802** which comprises a portion disposed in the sheath **626** and a portion outside of the sheath **626** which is connected to the processor **636.** The sensor well **620** is shown mated with one of the connectors **608** of the bioreactor vessel **632,** with the sheath **626** deployed into the interior of the vessel **632** so that the sensing element **628** is exposed to the media **634.** The signal transmitter **802** is in communication with processor **636,** which is configured to receive and interpret the signal from the sensing element **628.** The processor **636** can include a display portion **638** to display the information indicative of a characteristic or property of the media **634** sensed by the sensing element **628.**

To deploy the sensor well **620** in the bioreactor system **600,** a user can first select the appropriate pre-sterilized sensor well **620** for the particular application. The sensor well **620** can be removed from its packaging, and positioned such that the connector **624** is mated with the corresponding connector **608** of the bioreactor system **600.** Once the two connectors **624**, **608** are mated, any sealing layers provided on the connectors **624**, **608** can be removed, preferably simultaneously, to expose the pre-sterilized interior of the sensor well **620** to the aseptic interior of the bioreactor system **600.** Then, the proximal end of the sensor well **620** can be pressed toward the connectors **624**, **608** so as to insert the sensor well's sheath **626** into the interior of the bioreactor system **600.** Once deployed, the sensing element **628** is exposed to the media **634.** The sensor well **620** can optionally be locked into this deployed position, such that the sheath **626** is held in place contacting the media **634.** The state of the media **634** can thus be monitored using the sensor well **620,** and a user can determine what action (if any) to take with regard to the cell culture population in the bioreactor system **600** in response to information relayed from the sensing element **628.**

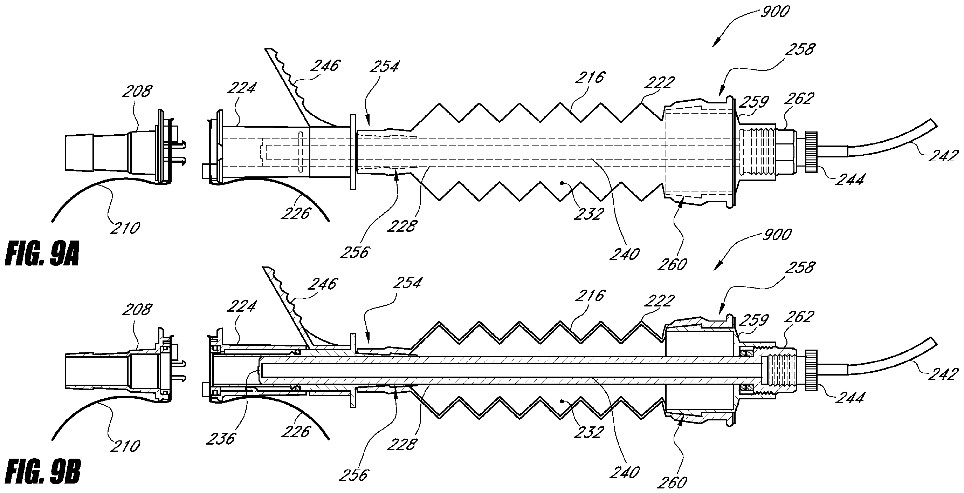

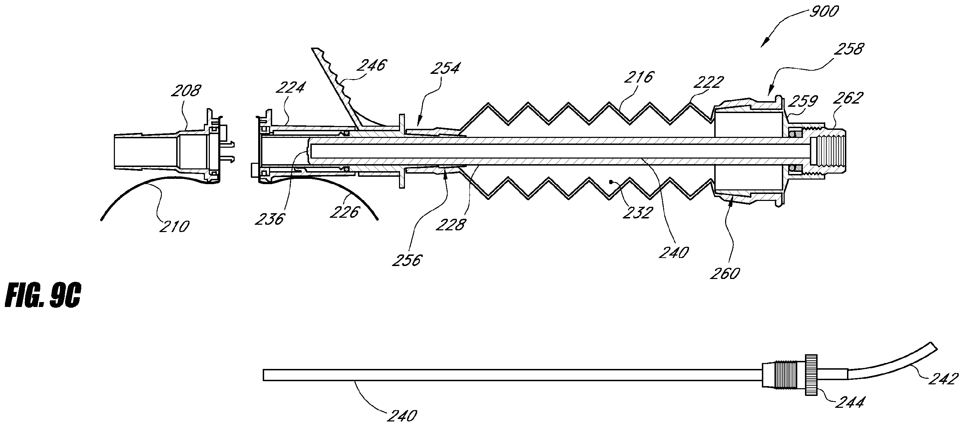

**FIGURES 9a, 9b****,** and **9c** illustrate a specific example of a sensor well **900** and show certain detail and structure in greater detail. This example contains some parts similar to those described above and are thus similarly labeled. These figures depict a sensor well **900** comprising an enclosure **216** with bellows **222,** a connector **224,** a sheath **228,** a sensing element **236,** and a signal transmitter **240.** The connector **224** can be attached to the enclosure **216** at the distal joint end **254** of the enclosure **216.** The proximal joint end **256** of the connector **224** can be inserted into the distal joint end **254** of the enclosure **216,** sealably connecting the two components. At the proximal joint end **258** of the enclosure **216** a threaded insert **259** can be sealably attached to the enclosure **216** by inserting the distal joint end **260** of the threaded insert **259** into the proximal joint end **258** of the enclosure **216.** A locking nut **262** which can be attached to the sheath **228** can then be screwed into the threaded insert **259** attaching the sheath **228** to the enclosure **216.** As depicted in the figures, the signal transmitter **240** can be inserted into the sheath **228** and secured by a support **244** which can be threaded into the sheath **228.** Each of these joints (or couplings), including but not limited to distal joint end **254** and proximal joint end **258** of the enclosure **216,** proximal joint end **256** of the connector **224,** and distal joint end **260** of the threaded insert **259,** may be further secured through the use of clamps, latches, adhesives, or any other known securement means.

The signal transmitter **240** is configured to provide signals to and/or receive signals from the sensing element **236** and provide signals to and/or receive signals from a sensor controller, or processor (not shown). The signal transmitter **240** is an optical waveguide. In another example, the signal transmitter **240** is a communication line or wire. In yet another example, a removable fiber optic assembly includes a signal transmitter **240,** a support **244,** and a communication line **242.** The removable fiber optic assembly can be inserted into the sheath **228.** In **FIGURE 9b**, for example, the signal transmitter **240** is shown inserted into the sheath **228.** In **FIGURE 9c**, the signal transmitter **240** is shown removed from the sheath **228.**

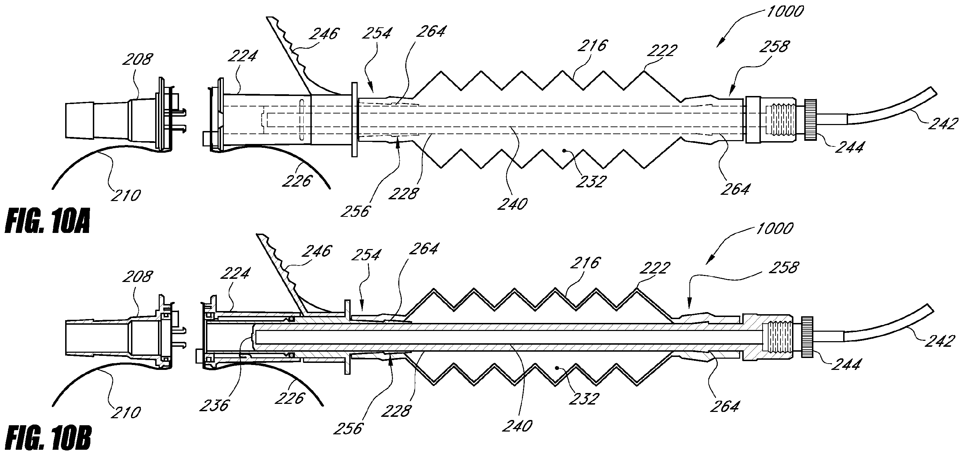

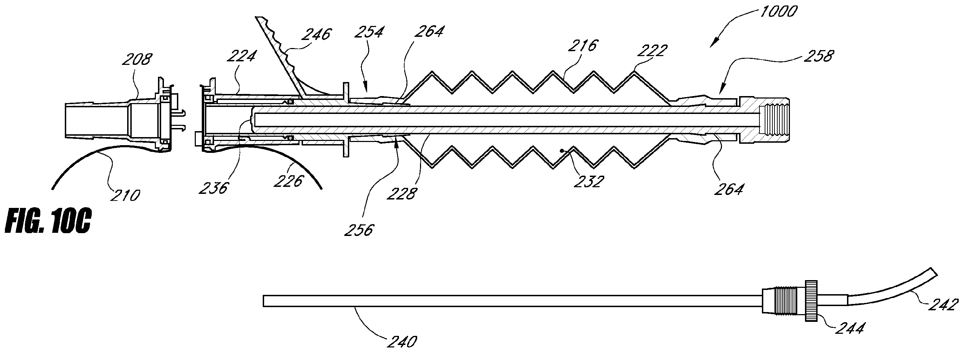

**FIGURES 10a, 10b****,** and **10c** illustrate another sensor well **1000.** These figures contain some parts similar to those described above and are thus similarly labeled. These figures depict a sensor well **1000** comprising an enclosure **216** with bellows **222,** a connector **224,** a sheath **228,** a sensing element **236,** and a signal transmitter **240.** The connector **224** can be attached to the enclosure **216** at the distal joint end **254** of the enclosure. The proximal joint end **256** of the connector **224** can be inserted into the distal joint end **254** of the enclosure **216** connecting the two components. This connection may be secured and sealed by heat fusing **264** the distal joint end **254** of the enclosure **216** to the proximal joint end **256** of the connector **224.** At the proximal joint end **258** of the enclosure **216** a sheath **228** can be sealably attached to the enclosure **216** by inserting the sheath **228** into the proximal joint end **258** of the enclosure **216.** This joint can be secured and sealed by heat fusing **264** the sheath **228** to the proximal joint end **258** of the enclosure **216.** As depicted in the figures, the signal transmitter **240** can be inserted into the sheath **228** and secured by a support **244** which can be threaded into the sheath **228.**

As described above with reference to **FIGURES 9a-9c**, the signal transmitter **240** is configured to provide signals to and/or receive signals from the sensing element **236** and provide signals to and/or receive signals from a sensor controller, or processor (not shown) The signal transmitter **240** is an optical waveguide. The signal transmitter **240** may include a communication line or wire. In one example, the signal transmitter **240** includes a wire that provides a signal to an optical element that irradiates the sensing element, and/or receives a signal from a detector that receives illumination from the sensing element 236. In yet another example, a removable fiber optic assembly includes a signal transmitter **240,** a support **244,** and a communication line **242.** The removable fiber optic assembly can be inserted into the sheath **228.** In **FIGURE 10b**, for example, the signal transmitter **240** is shown inserted into the sheath **228.** In **FIGURE 10c**, the signal transmitter **240** is shown removed from the sheath **228.**

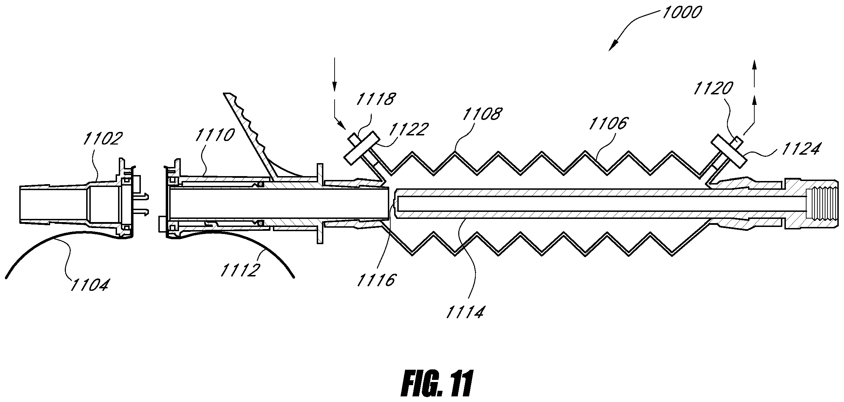

**FIGURE 11** illustrates an embodiment according to the invention of a sensor well **1100.** The sensor well **1100** in this embodiment can be attached to a sterile connector **1102** which can have a removable seal **1104** and can be incorporated into a bioreactor system. The sensor well **1100** comprises a sterilizable enclosure **1106** which may have bellows **1108,** a sterile connector **1110** which can have a removable seal **1112,** a sheath **1114** which can be attached to the enclosure **1106,** and a sensing element **1116** which can be located on the tip of the sheath **1114.** This embodiment further comprises an inlet port, or calibration gas inlet **1118,** an outlet port, or calibration gas outlet **1120,** and a first **1122** and second **1124** submicron filter. The inlet and outlet ports **1118, 1120** can allow the passage of a sterile gas through the sealed enclosure **1106.** This gas can be cleansed by the first **1122** and second **1124** submicron filters. This gas can be used to zero and/or calibrate the sensing element **1116** or to verify the calibration data for the sensing element **1116** without disturbing the aseptic condition of the enclosure **1106.** The inlet **1118** and outlet **1120** ports can be formed as part of the enclosure **1106,** or can be formed as separate components and then attached to the sensor well **1100.**

Although illustrated within the context of a disposable bioreactor system, embodiments of the present invention may also be used with other containers and systems for which an ability to monitor media contained therein, while maintaining a sterile environment therein, is desirable. In addition, it will be understood by those of skill in the art that numerous and various modifications can be made without departing from the scope of the present invention as defined by the claims. Therefore, it should be clearly understood that the forms of the invention described herein are illustrative only and are not intended to limit the scope of the invention. In addition, it will be understood that various omissions, substitutions, and changes in the form and details of the device or process illustrated may be made by those skilled in the art without departing from the spirit of the invention. As will be recognized, the present invention may be embodied within a form that does not provide all of the features and benefits set forth herein, as some features may be used or practiced separately from others.

### SPECIFIC EMBODIMENTS

Specific embodiments and description follow.

The present invention relates to a sensor well for use with a bioreactor vessel comprising
a sheath (116, 228) having proximal end (118, 230) and a distal end (120, 238), wherein the sheath is enclosed at the distal end,
a sensing element (126, 236) comprising a fluorescing material disposed on the distal end of the sheath (116, 228),
a signal transmitter (127, 240) comprising an optical waveguide disposed within at least a portion of the sheath (116, 228), one end of the signal transmitter (127, 240) located at the distal end of the sheath (116, 228), the signal transmitter (127, 240) positioned to transmit light from a sensor controller to illuminate the sensing element (126, 236), the optical waveguide configured to receive illumination signals from the sensing element (126, 236) and provide signals from the sensing element (126, 236) to a sensor controller,
a connector (110, 222) comprising an aperture, the connector configured to attach the sensor well to a portion of a bioreactor vessel;
and a collapsible bellows (114, 222) coupled on one end to the connector and on the other end to the proximal end of the sheath (116, 228), bellows (114, 222) housing the sheath and the sending element on the end of the sheath when the sheath is in an undeployed position not extending through the aperture in the connector (110, 208), wherein the bellows (114, 222), the connector (110, 208), and the sheath (116, 228) are connected to form at least a portion of a hermetically sealable and sterilizable enclosure in which the sensing element is enclosed when the sheath is in the undeployed position,
a removable sealing layer (112, 226) covering the distal end of the sterilizable enclosure (102, 216) configured to hermetically seal the sterilizable enclosure (102, 216),
a calibration gas inlet (1118) with a first (1122) submicron filter and a calibration gas outlet (1120) with a second (1124) submicron filter configured to allow passage of a gas through the enclosure to calibrate the fluorescing material enclosed in the enclosure, wherein the sheath (116, 228) and the bellows (114, 222) are configured so that the sheath (116, 228) can be deployed through the aperture in the connector (110, 208) and into a bioreactor vessel that is attached to the connector thereby exposing the sensing element (126, 236) on the distal end of the sheath (116, 228) to media contained in the bioreactor vessel while maintaining a sterile environment in the bioreactor vessel, and
the sensor well is provided pre-sterilized and sealed within a sterile package (250).

The fluorescing material can be configured as a fluorescent dot configured to fluoresce, when radiated, to indicate a characteristic of a cell culture population to which it is exposed. The sensor well (100, 200) can further comprise a plurality of sensing elements (126, 236) and a plurality of signal transmitters (127, 240), each signal transmitter (127, 240) associated with at least one sensing element (126, 236). The sensing element can comprise an electronic sensor configured to sense one of temperature, conductivity, and osmolality. The fluorescing material can be configured to indicate one of pH, dissolved oxygen content, and carbon dioxide content. The bellows can comprise a telescoping structure. The bellows can comprise a flexible material configured in an accordion-like structure. The fluorescing material can be configured to indicate pH. The fluorescing material can be configured to indicate dissolved oxygen content. The fluorescing material can be configured to indicate carbon dioxide content. The proximal end of the sheath can comprise an aperture configured to accept the signal transmitter (127, 240).

Described herein a device for use with a bioreactor system comprises a waveguide; a sheath surrounding the waveguide; a sensing element disposed at a distal end of the sheath, the sheath and sensing element configured to move between a first position and a second position; a sterilizable enclosure configured to protect the sensing element from an exterior environment at least when the sheath and the sensing element are in the first position; and a sterile connector disposed at a distal end of the enclosure, wherein the distal end of the sheath is disposed distal of the sterile connector when the sheath and the sensing element are in the second position. The sensing element comprises a fluorescing material. The sensing element can comprise one or more dots each comprising fluorescing material. At least one of the dots can be configured to fluoresce, when radiated, to indicate a characteristic of a cell culture population to which it is exposed. One or more dots can be configured to fluoresce to indicate pH. One or more dots can be configured to fluoresce to indicate dissolved oxygen content. One or more dots can be configured to fluoresce to indicate carbon dioxide content. The sensing element can be an electronic sensor. The sensing element can be configured to measure temperature. The sensing element can be configured to measure conductivity. The sensing element can be configured to measure osmolality.

Described herein a device for use with a bioreactor vessel comprises a sensor comprising a signal transmitter, a sheath, the sheath at least partially surrounding the signal transmitter, and at least one sensing element disposed at a distal end of the sheath, the sensor configured to move between a first and a second position; bellows surrounding at least a portion of the sensor at least when the sensor is in the first position; and a sterile connector disposed at a distal end of the bellows, wherein the bellows, the sterile connector, and the sheath form a sterilizable enclosure when the sensor is in the first position, and wherein the sensor is movable with respect to the connector such that the distal end of the sheath is disposed distal of the sterile connector when the sensor is in the second position. The signal transmitter comprises an optical waveguide and the sensing element comprises a fluorescent dot. The signal transmitter comprises a communication wire.

Described herein a device for use with a bioreactor vessel comprises a sensor comprising at least one signal transmitter and at least one sensing element; and an enclosure defining a sterilizable space around the sensor, the enclosure comprising a connector configured to provide a sterile connection between the enclosure and a corresponding connector on a bioreactor vessel.

Described herein a device for monitoring media in a bioreactor bag having at least one port with a first sterile connector coupled thereto comprises means for monitoring a characteristic of the media, the monitoring means comprising at least one fluorescent dot, means for transmitting light to the fluorescent dot, and means for transmitting a response signal from the fluorescent dot to a controller; means for protecting a sterile environment of the monitoring means; means for making a sterile connection between the monitoring means and the first sterile connector; and means for inserting the monitoring means into the bioreactor bag once a sterile connection has been made. The means for transmitting light can comprise an optical waveguide. The means for transmitting a response signal from the fluorescent dot to the controller can comprise an optical waveguide.

Described herein a bioreactor system comprises a disposable bioreactor bag comprising at least one port having a first sterile connector coupled thereto; a sensor comprising at least one signal transmitter, at least one sensing element, and a second sterile connector; and an enclosure defining a sterilizable space around at least the sensing element, the enclosure comprising a connector configured to provide a sterile connection between the enclosure and a corresponding connector on a bioreactor bag.

The present invention also provides a method of monitoring media in a bioreactor vessel comprising:
removing a pre-sterilized sterile disposable sensor well (200) from a sterile package (250);
coupling a first sterile connector (208) of the bioreactor vessel to a second sterile connector (224) of a disposable sensor well, the sensor well comprising bellows (222) and a moveable member, the bellows, the moveable member, a seal (226) of the second sterile connector (224), and the second sterile connector forming a sterilizable space therebetween, the injectable member comprising at least one waveguide (240) disposed inside the movable member and at least one fluorescent dot on an end of the movable member, the end of the moveable member being disposed in the sterilizable space before the moveable member is moved into the bioreactor vessel, the sensor well (200) further comprising a calibration gas inlet (1118) with a first (1122) submicron filter and a calibration gas outlet (1120) with a second (1124) submicron filter configured to allow passage of a gas through the enclosure to calibrate the fluorescing material enclosed in the enclosure;
removing the seal of the second sterile connector;
moving the injectable member into the bioreactor vessel (202) to expose the fluorescent dot to the inside of the bioreactor vessel;
transmitting, through the at least one waveguide (240), a particular wavelength of light from a sensor controller to illuminate the at least one fluorescent dot;
receiving an illumination signal from the at least one fluorescent dot; and
providing the received illumination signal from the at least one fluorescent dot to the sensor controller using the at least one waveguide.

## Claims

1. A sensor well (100, 200) for use with a bioreactor vessel, the sensor well comprising:
a sheath (116, 228) having proximal end (118, 230) and a distal end (120, 238), wherein the sheath is enclosed at the distal end;
a sensing element (126, 236) comprising a fluorescing material disposed on the distal end of the sheath (116, 228);
a signal transmitter (127, 240) comprising an optical waveguide disposed within at least a portion of the sheath (116, 228), one end of the signal transmitter (127, 240) located at the distal end of the sheath (116, 228), the signal transmitter (127, 240) positioned to transmit light from a sensor controller to illuminate the sensing element (126, 236), the optical waveguide configured to receive illumination signals from the sensing element (126, 236) and provide signals from the sensing element (126, 236) to the sensor controller;
a connector (110, 208) comprising an aperture, the connector configured to attach the sensor well to a portion of a bioreactor vessel; and
a collapsible bellows (114, 222) coupled on one end to the connector and on the other end to the proximal end of the sheath (116, 228), the bellows (114, 222) housing the sheath and the sensing element on the end of the sheath when the sheath is in an undeployed position not extending through the aperture in the connector (110, 208), wherein the bellows (114, 222), the connector (110, 208), and the sheath (116, 228) are connected to form at least a portion of a hermetically sealable and sterilizable enclosure (102, 216) in which the sensing element is enclosed when the sheath is in the undeployed position,
a removable sealing layer (112, 226) covering the distal end of the sterilizable enclosure (102, 216) configured to hermetically seal the sterilizable enclosure (102, 216),
a calibration gas inlet (1118) with a first (1122) submicron filter and a calibration gas outlet (1120) with a second (1124) submicron filter configured to allow passage of a gas through the enclosure to calibrate the fluorescing material enclosed in the enclosure,
wherein the sheath (116, 228) and the bellows (114, 222) are configured so that the sheath (116, 228) can be deployed through the aperture in the connector (110, 208) and into a bioreactor vessel that is attached to the connector thereby exposing the sensing element (126, 236) on the distal end of the sheath (116, 228) to media contained in the bioreactor vessel while maintaining a sterile environment in the bioreactor vessel, and
the sensor well is provided pre-sterilized and sealed within a sterile package (250).

2. The sensor well (100, 200) of claim 1, wherein the fluorescing material comprises a fluorescent dot configured to fluoresce, when radiated, to indicate a characteristic of a cell culture population to which it is exposed.

3. The sensor well (100, 200) of claim 1, further comprising a plurality of sensing elements (126, 236) and a plurality of signal transmitters (127, 240), each signal transmitter (127, 240) associated with at least one sensing element (126, 236).

4. The sensor well (100, 200) of claim 1, wherein the sensing element (126, 236) further comprises an electronic sensor configured to sense one of temperature, conductivity, and osmolality.

5. The sensor well (100, 200) of any of claims 1 to 4, wherein the fluorescing material is configured to indicate one of pH, dissolved oxygen content, and carbon dioxide content.

6. The sensor well (100, 200) of claim 1, wherein the sensing element (126,236) comprises a plurality of fluorescent dots of fluorescent material configured to fluoresce to indicate one more of pH, dissolved oxygen content, and carbon dioxide content.

7. The sensor well (100, 200) of any of claims 1 to 6, wherein the proximal end of the sheath comprises an aperture configured to accept the signal transmitter (127, 240).

8. A method of monitoring media in a bioreactor vessel (202), the method comprising:
removing a pre-sterilized sterile disposable sensor well (200) from a sterile package (250),
coupling a first sterile connector (208) of the bioreactor vessel to a second sterile connector (224) of the disposable sensor well (200), the sensor well comprising bellows (222) and a movable member, the bellows (222), the movable member, a seal (226) of the second sterile connector (224), and the second sterile connector forming a sterilizable space therebetween, the movable member comprising at least one waveguide (240) disposed inside the movable member and at least one fluorescent dot on an end of the movable member, the end of the movable member disposed in the sterilizable space before the movable member is moved into the bioreactor vessel (202), the sensor well (200) further comprising a calibration gas inlet (1118) with a first (1122) submicron filter and a calibration gas outlet (1120) with a second (1124) submicron filter configured to allow passage of a gas through the sterile enclosure of the sensor well (200) to calibrate the fluorescing material enclosed in the sterile enclosure;
removing the seal (226) of the second sterile connector;
moving the movable member into the bioreactor vessel (202) to expose the fluorescent dot to the inside of the bioreactor vessel;
transmitting, through the at least one waveguide (240), a particular wavelength of light from a sensor controller to illuminate the at least one fluorescent dot;
receiving an illumination signal from the at least one fluorescent dot; and
providing the received illumination signal from the at least one fluorescent dot to the sensor controller using the at least one waveguide.

9. The method of claim 8, wherein the sensor well comprises the sensor well of claim 1.

10. The method of claim 8 or 9, wherein providing signals from the at least one fluorescent dot comprises providing information to the sensor controller regarding one of pH, dissolved oxygen content, and carbon dioxide content.

11. The method of any of claims 8 to 10, further comprising measuring with an electronic sensor one of temperature, conductivity, and osmolality of the media.

12. The method of any of claims 8 to 11, further comprising determining an analyte concentration of the media in the bioreactor vessel based on the signal provided signal from the at least one fluorescent dot.

## Patentansprüche

1. Sensorkammer (100, 200) zur Verwendung mit einem Bioreaktorgefäß, wobei die Sensorkammer umfasst:
eine Hülle (116, 228) mit einem proximalen Ende (118, 230) und einem distalen Ende (120, 238), wobei die Hülle an dem distalen Ende geschlossen ist;
ein Sensorelement (126, 236), das ein Fluoreszenzmaterial umfasst, an dem distalen Ende der Hüllen (116, 228) angeordnet;
einen Signalüberträger (127, 240), der einen optischen Wellenleiter umfasst, innerhalb wenigstens eines Teils der Hüllen (116, 228) angeordnet, wobei ein Ende des Signalüberträgers (127, 240) an dem distalen Ende der Hüllen (116, 228) angeordnet ist, wobei der Signalüberträger (127, 240) angeordnet ist, Licht von einer Sensorsteuerung zu übertragen, um das Sensorelement (126, 236) zu beleuchten, wobei der optische Wellenleiter dafür gestaltet ist, Beleuchtungssignale von dem Sensorelement (126, 236) zu empfangen und der Sensorsteuerung Signale von dem Sensorelement (126, 236) zu liefern;
ein Verbindungselement (110, 208), das eine Öffnung aufweist, wobei das Verbindungselement dafür gestaltet ist, die Sensorkammer an einem Teil eines Bioreaktorgefäßes zu befestigen; und
einen kollabierbaren Balg (114, 222), der an einem Ende an das Verbindungselement und an dem anderen Ende an das proximale Ende der Hüllen (116, 228) gekoppelt ist, wobei der Balg (114, 222) die Hülle und das Sensorelement an dem Ende der Hüllen enthält, wenn die Hülle in einer nicht ausgefahrenen Stellung vorliegt, die nicht durch die Öffnung in dem Verbindungselement (110, 208) ragt, wobei der Balg (114, 222), das Verbindungselement (110, 208) und die Hülle (116, 228) verbunden sind, um wenigstens einen Teil einer hermetisch andichtbaren und sterilisierbaren Umhüllung (102, 216) zu bilden, in der das Sensorelement eingeschlossen ist, wenn sich die Hülle in der nicht ausgefahrenen Stellung befindet,
eine entfernbare Abdichtungsschicht (112, 226), die das distale Ende der sterilisierbaren Umhüllung (102, 216) bedeckt, dafür gestaltet, die sterilisierbare Umhüllung (102, 216) hermetisch abzudichten,
einen Kalibriergaseinlass (1118) mit einem ersten (1122) Submikrometerfilter und einen Kalibriergasauslass (1120) mit einem zweiten (1124) Submikrometerfilter, dafür gestaltet, das Durchtreten eines Gases durch die Umhüllung zu erlauben, um das in der Umhüllung eingeschlossene Fluoreszenzmaterial zu kalibrieren,
wobei der Hüllen (116, 228) und der Balg (114, 222) so gestaltet sind, dass der Hüllen (116, 228) durch die Öffnung in dem Verbindungselement (110, 208) und in ein an dem Verbindungselement befestigtes Bioreaktorgefäß ausgefahren werden kann, um das Sensorelement (126, 236) an dem distalen Ende der Hüllen (116, 228) gegenüber einem in dem Bioreaktor enthaltenen Medium zu exponieren, wobei eine sterile Umgebung in dem Bioreaktorgefäß erhalten bleibt, und
die Sensorkammer vorsterilisiert und abgedichtet in einer sterilen Verpackung (250) bereitgestellt wird.

2. Sensorkammer (100, 200) gemäß Anspruch 1, wobei das Fluoreszenzmaterial einen Fluoreszenzpunkt aufweist, der dafür gestaltet ist, wenn bestrahlt, zu fluoreszieren, um ein Merkmal einer Zellkulturpopulation, gegenüber der er exponiert ist, anzuzeigen.

3. Sensorkammer (100, 200) gemäß Anspruch 1, ferner umfassend eine Vielzahl von Sensorelementen (126, 236) und eine Vielzahl von Signalüberträgern (127, 240), wobei jeder Signalüberträger (127, 240) mit wenigstens einem Sensorelement (126, 236) verbunden ist.

4. Sensorkammer (100, 200) gemäß Anspruch 1, wobei das Sensorelement (126, 236) ferner einen elektronischen Sensor umfasst, der dafür gestaltet ist, eines von Temperatur, Leitfähigkeit und Osmolalität zu erfassen.

5. Sensorkammer (100, 200) gemäß einem der Ansprüche 1 bis 4, wobei das Fluoreszenzmaterial dafür gestaltet ist, eines von pH-Wert, Gehalt an gelöstem Sauerstoff und Kohlendioxidgehalt anzuzeigen.

6. Sensorkammer (100, 200) gemäß Anspruch 1, wobei das Sensorelement (126, 236) eine Vielzahl von Fluoreszenzpunkten umfasst, die dafür gestaltet sind, zu fluoreszieren, um eines oder mehrere von pH-Wert, Gehalt an gelöstem Sauerstoff und Kohlendioxidgehalt anzuzeigen.

7. Sensorkammer (100, 200) gemäß einem der Ansprüche 1 bis 6, wobei das proximale Ende der Hüllen eine Öffnung umfasst, die dafür gestaltet ist, den Signalüberträger (127, 240) aufzunehmen.

8. Verfahren zur Überwachung eines Mediums in einem Bioreaktorgefäß (202), wobei das Verfahren umfasst:
Entnehmen einer vorsterilisierten sterilen wegwerfbaren Sensorkammer (200) aus einer sterilen Verpackung (250),
Koppeln eines ersten sterilen Verbindungselements (208) des Bioreaktorgefäßes an ein zweites steriles Verbindungselement (224) der wegwerfbaren Sensorkammer (200), wobei die Sensorkammer einen Balg (222) und ein bewegliches Element umfasst, wobei der Balg (222), das bewegliche Element, eine Abdichtung (226) des zweiten sterilen Verbindungselements (224) und das zweite sterile Verbindungselement einen sterilisierbaren Raum zwischen sich bilden, wobei das bewegliche Element wenigstens einen Wellenleiter (240), der innerhalb des beweglichen Elements angeordnet ist, und wenigstens einen Fluoreszenzpunkt an einem Ende des beweglichen Elements umfasst, wobei das Ende des beweglichen Elements in dem sterilisierbaren Raum angeordnet ist, bevor das bewegliche Element in das Bioreaktorgefäß (202) bewegt wird, wobei die Sensorkammer (200) ferner einen Kalibriergaseinlass (1118) mit einem ersten (1122) Submikrometerfilter und einen Kalibriergasauslass (1120) mit einem zweiten (1124) Submikrometerfilter umfasst, dafür gestaltet, das Durchtreten eines Gases durch die sterile Umhüllung der Sensorkammer (200) zu erlauben, um das in der sterilen Umhüllung eingeschlossene Fluoreszenzmaterial zu kalibrieren;
Entfernen der Abdichtung (226) des zweiten sterilen Verbindungselements;
Bewegen des beweglichen Elements in das Bioreaktorgefäß (202), um den Fluoreszenzpunkt gegenüber dem Innenraum des Bioreaktorgefäßes zu exponieren;
durch den wenigstens einen Wellenleiter (240) Übertragen einer bestimmten Lichtwellenlänge von einer Sensorsteuerung, um den wenigstens einen Fluoreszenzpunkt zu beleuchten;
Empfangen eines Beleuchtungssignals von dem wenigstens einen Fluoreszenzpunkt; und
durch den wenigstens einen Wellenleiter Liefern des empfangenen Beleuchtungssignals von dem wenigstens einen Fluoreszenzpunkt an die Sensorsteuerung.

9. Verfahren gemäß Anspruch 8, wobei die Sensorkammer eine Sensorkammer gemäß Anspruch 1 umfasst.

10. Verfahren gemäß Anspruch 8 oder 9, wobei das Liefern von Signalen von dem wenigstens einen Fluoreszenzpunkt das Liefern von Informationen hinsichtlich einem von pH-Wert, Gehalt an gelöstem Sauerstoff und Kohlendioxidgehalt an die Sensorsteuerung umfasst.

11. Verfahren gemäß einem der Ansprüche 8 bis 10, ferner umfassend Messen von einem von Temperatur, Leitfähigkeit und Osmolalität mithilfe eines elektronischen Sensors.

12. Verfahren gemäß einem der Ansprüche 8 bis 11, ferner umfassend Bestimmen einer Analytkonzentration des Mediums in dem Bioreaktorgefäß auf der Grundlage des von dem wenigstens einen Fluoreszenzpunkt gelieferten Signals.

## Revendications

1. Doigt de gant de capteur (100, 200) à utiliser avec une cuve de bioréacteur, le doigt de gant de capteur comprenant :
une gaine (116, 228) ayant une extrémité proximale (118, 230) et une extrémité distale (120, 238), la gaine étant fermée à l'extrémité distale ;
un élément de détection (126, 236) comprenant un matériau fluorescent disposé sur l'extrémité distale de la gaine (116, 228) ;
un émetteur de signaux (127, 240) comprenant un guide d'ondes optiques disposé à l'intérieur d'au moins une partie de la gaine (116, 228), une extrémité de l'émetteur de signaux (127, 240) étant située à l'extrémité distale de la gaine (116, 228), l'émetteur de signaux (127, 240) étant positionné pour émettre de la lumière depuis un contrôleur de capteur pour éclairer l'élément de détection (126, 236), le guide d'ondes optiques étant configuré pour recevoir des signaux d'éclairage provenant de l'élément de détection (126, 236) et fournir des signaux provenant de l'élément de détection (126, 236) au contrôleur de capteur ;
un connecteur (110, 208) comprenant une ouverture, le connecteur étant configuré pour attacher le doigt de gant de capteur à une partie d'une cuve de bioréacteur ; et
un soufflet pliable (114, 222) couplé sur une extrémité au connecteur et sur l'autre extrémité à l'extrémité proximale de la gaine (116, 228), le soufflet (114, 222) accueillant la gaine et l'élément de détection sur l'extrémité de la gaine quand la gaine est dans une position non déployée ne s'étendant pas à travers l'ouverture dans le connecteur (110, 208), le soufflet (114, 222), le connecteur (110, 208) et la gaine (116, 228) étant raccordés pour former au moins une partie d'une enceinte scellable hermétiquement et stérilisable (102, 216) dans laquelle l'élément de détection est enfermé quand la gaine est dans la position non déployée,
une couche de scellement amovible (112, 226) recouvrant l'extrémité distale de l'enceinte stérilisable (102, 216) configurée pour sceller hermétiquement l'enceinte stérilisable (102, 216),
une entrée de gaz d'étalonnage (1118) avec un premier filtre submicronique (1122) et une sortie de gaz d'étalonnage (1120) avec un deuxième filtre submicronique (1124), configurées pour permettre le passage d'un gaz à travers l'enceinte pour étalonner le matériau fluorescent enfermé dans l'enceinte,
dans lequel la gaine (116, 228) et le soufflet (114, 222) sont configurés de telle sorte que la gaine (116, 228) peut être déployée à travers l'ouverture dans le connecteur (110, 208) et à l'intérieur d'une cuve de bioréacteur qui est attachée au connecteur pour exposer ainsi l'élément de détection (126, 236) sur l'extrémité distale de la gaine (116, 228) à un milieu contenu dans la cuve de bioréacteur tout en maintenant un environnement stérile dans la cuve de bioréacteur, et
le doigt de gant de capteur est fourni préstérilisé et scellé à l'intérieur d'un emballage stérile (250).

2. Doigt de gant de capteur (100, 200) de la revendication 1, dans lequel le matériau fluorescent comprend un point fluorescent configuré pour fluorescer, lorsqu'il est irradié, pour indiquer une caractéristique d'une population de culture cellulaire à laquelle il est exposé.

3. Doigt de gant de capteur (100, 200) de la revendication 1, comprenant en outre une pluralité d'éléments de détection (126, 236) et une pluralité d'émetteurs de signaux (127, 240), chaque émetteur de signaux (127, 240) étant associé à au moins un élément de détection (126, 236).

4. Doigt de gant de capteur (100, 200) de la revendication 1, dans lequel l'élément de détection (126, 236) comprend en outre un capteur électronique configuré pour détecter un paramètre parmi la température, la conductivité, et l'osmolalité.

5. Doigt de gant de capteur (100, 200) de l'une quelconque des revendications 1 à 4, dans lequel le matériau fluorescent est configuré pour indiquer un paramètre parmi le pH, la teneur en oxygène dissous, et la teneur en dioxyde de carbone.

6. Doigt de gant de capteur (100, 200) de la revendication 1, dans lequel l'élément de détection (126, 236) comprend une pluralité de points fluorescents de matériau fluorescent configurés pour fluorescer pour indiquer un ou plusieurs paramètres parmi le pH, la teneur en oxygène dissous, et la teneur en dioxyde de carbone.

7. Doigt de gant de capteur (100, 200) de l'une quelconque des revendications 1 à 6, dans lequel l'extrémité proximale de la gaine comprend une ouverture configurée pour accepter l'émetteur de signaux (127, 240) .

8. Procédé de surveillance d'un milieu dans une cuve de bioréacteur (202), le procédé comprenant :
le retrait d'un doigt de gant de capteur jetable stérile préstérilisé (200) d'un emballage stérile (250),
le couplage d'un premier connecteur stérile (208) de la cuve de bioréacteur à un deuxième connecteur stérile (224) du doigt de gant de capteur jetable (200), le doigt de gant de capteur comprenant un soufflet (222) et un élément mobile, le soufflet (222), l'élément mobile, un joint d'étanchéité (226) du deuxième connecteur stérile (224) et le deuxième connecteur stérile formant entre eux un espace stérilisable, l'élément mobile comprenant au moins un guide d'ondes (240) disposé à l'intérieur de l'élément mobile et au moins un point fluorescent sur une extrémité de l'élément mobile, l'extrémité de l'élément mobile étant disposée dans l'espace stérilisable avant que l'élément mobile soit déplacé à l'intérieur de la cuve de bioréacteur (202), le doigt de gant de capteur (200) comprenant en outre une entrée de gaz d'étalonnage (1118) avec un premier filtre submicronique (1122) et une sortie de gaz étalonnage (1120) avec un deuxième filtre submicronique (1124), configurées pour permettre le passage d'un gaz à travers l'enceinte stérile du doigt de gant de capteur (200) pour étalonner le matériau fluorescent enfermé dans l'enceinte stérile ;
le retrait du joint d'étanchéité (226) du deuxième connecteur stérile ;
le déplacement de l'élément mobile à l'intérieur de la cuve de bioréacteur (202) pour exposer le point fluorescent à l'intérieur de la cuve de bioréacteur ;
l'émission, par le biais de l'au moins un guide d'ondes (240), d'une longueur d'onde particulière de lumière depuis un contrôleur de capteur pour éclairer l'au moins un point fluorescent ;
la réception d'un signal d'éclairage provenant de l'au moins un point fluorescent ; et
la fourniture du signal d'éclairage reçu depuis l'au moins un point fluorescent au contrôleur de capteur au moyen de l'au moins un guide d'ondes.

9. Procédé de la revendication 8, dans lequel le doigt de gant de capteur comprend le doigt de gant de capteur de la revendication 1.

10. Procédé de la revendication 8 ou 9, dans lequel la fourniture de signaux depuis l'au moins un point fluorescent comprend la fourniture au contrôleur de capteur d'informations concernant un paramètre parmi le pH, la teneur en oxygène dissous, et la teneur en dioxyde de carbone.

11. Procédé de l'une quelconque des revendications 8 à 10, comprenant en outre la mesure avec un capteur électronique d'un paramètre parmi la température, la conductivité et l'osmolalité du milieu.

12. Procédé de l'une quelconque des revendications 8 à 11, comprenant en outre la détermination d'une concentration d'analyte du milieu dans la cuve de bioréacteur sur la base du signal fourni depuis l'au moins un point fluorescent.
